## Europäisches Patentamt
## European Patent Office
## Office européen des brevets

(19)

(11) Publication number: **0 019 341**
**B1**

(12) **EUROPEAN PATENT SPECIFICATION**

(45) Date of publication of patent specification: **25.05.83**

(21) Application number: **80200444.0**

(22) Date of filing: **13.05.80**

(51) Int. Cl.³: **C 07 C 63/08,**
**C 07 C 33/22,**
**C 07 C 27/02,**
**C 07 C 69/78, C 07 C 67/60**

(54) Method for the preparation of an alkali metal benzoate together with benzyl alcohol.

(30) Priority: **17.05.79 NL 7903876**

(43) Date of publication of application:
**26.11.80 Bulletin 80/24**

(45) Publication of the grant of the patent:
**25.05.83 Bulletin 83/21**

(84) Designated Contracting States:
**BE DE FR GB IT NL**

(56) References cited:
**EP - A - 0 005 574**
**DE - A - 2 743 004**
**DE - B - 1 003 709**
**GB - A - 1 317 342**

(73) Proprietor: **STAMICARBON B.V.**
**Postbus 10**
**NL-6160 MC Geleen (NL)**

(72) Inventor: **Jongsma, Cornelis**
**Drossaertweide 1**
**NL-6438 HS Oirsbeek (NL)**
Inventor: **Frijns, Léon Hubertus Barbara**
**Cremerstraat 13**
**NL-6301 GD Valkenburg (NL)**
Inventor: **Raven-Donners, Paula Arnolda Maria**
**Dr. Nolenslaan 8**
**NL-6136 GN Sittard (NL)**

(74) Representative: **Hoogstraten, Willem Cornelis**
**Roeland et al,**
**OCTROOIBUREAU DSM Postbus 9**
**NL-6160 MA Geleen (NL)**

Courier Press, Leamington Spa, England.

# Method for the preparation of an alkali metal benzoate together with benzyl alcohol

The invention relates to a method for the preparation of an alkali metal benzoate together with benzyl alcohol from benzyl benzoate prepared by oxidation of toluene, with a gas containing molecular oxygen.

It is known from the German patent application DE—A—2743004 to subject the product obtained by oxidation of toluene by means of air in the presence of cobaltous acetate and manganese acetate and distillation of benzoic acid, to hydrogenation and to recover benzoic acid from the hydrogenation product (example 1).

Sodium benzoate is an important substance, which is applied, e.g., as a preservative in the food industry. Such a use often requires a high degree of purity of the sodium benzoate. However, crude sodium benzoate prepared from reaction products of the oxidation of toluene with a gas containing molecular oxygen, contains cumbersome impurities which are very hard to remove. Specifically, it is difficult to obtain from crude sodium benzoate a product which meets the pharmacopoeial requirements.

The purpose of the invention is to provide a solution to this problem. According to the invention crude benzyl benzoate, obtained as a by-product after distillation of benzoic acid from a reaction mixture prepared by oxidation of toluene with a gas containing molecular oxygen, is subjected to a treatment of 290—380K with molecular hydrogen or at 270—380K with a metal with reducing properties from the groups IA, IIA, IIB, IIIA, IVA, VIIB and VIII of the periodic system in the presence of water under such circumstances that there will be no conversion of any substantial quantity of benzyl benzoate, which treatment is preferably followed by a washing with an aqueous alkaline solution. Preferably the benzyl benzoate is then distilled. Subsequently the benzyl benzoate is saponified, for instance with an alkali metal hydroxide solution, to the alkali metal benzoate and benzyl alcohol.

From the resulting solution the benzyl alcohol can be extracted with, e.g., an organic extractant, such as toluene, and the alcohol can subsequently be recovered by distillation. The alkali metal benzoate can, e.g., be recovered by evaporation of the aqueous solution obtained.

Preferably this solution is cleaned, before this evaporation, of residues of toluene, benzyl alcohol, etc., e.g. by steam distillation.

The alkali metal benzoate obtained, specifically the sodium benzoate, meets the said pharmacopoeial requirements, which, as far as sodium benzoate is concerned, mean that, after drying to constant weight, it must be capable, in the following tests, of achieving the stated results:

1. acidity/alkalinity—2 g dissolved in water must not require more than 0.05—0.15 ml 0.1 N aqueous NaOH/HCl for neutralization (indicator: phenolphthalein).
2. $KMnO_4$ number—1 g sodium benzoate in acid medium must not require more than 0.5 ml 0.1 N $KMnO_4$.
3. content—percentage by weight of sodium benzoate must be at least 99.5 %.
4. colour—a solution of 10 g in 100 ml $H_2O$ must be of a lighter colour than or of the same colour as a solution in 100 ml 1 N aqueous sulphuric acid of 4 ml colour standard as described in Nederlandse Farmacopee VI.
5. turbidity—a solution of 10 g in 100 ml $H_2O$ must be clearer than a suspension in 100 ml $H_2O$ of 1 ml standard suspension of Bolus Alba as described in Nederlandse Farmacopee VI.
6. $H_2SO_4$ test—the colour of 0.5 g sodium benzoate mixed with 5 ml concentrated $H_2SO_4$ must, after 15 minutes, not be more intense than the standard solution "Matching Fluid Q" (MFQ) as described in US Pharmacopeia XIX.

Suitable reducing substances that can be used in applying the method according to the invention are hydrogen, which is preferably used in the presence of a suitable hydrogenation catalyst, and the metals with reducing properties from the groups IA, IIA, IIB, IIIA, IVA, VIIB and VIII of the periodic system, e.g. magnesium, calcium and in particular zinc, iron and aluminium, which are used in the presence of water, e.g. by bringing them, in the form of an aqueous suspension, into contact with the benzyl benzoate.

Suitable hydrogenation catalysts in the treatment of the benzyl benzoate with hydrogen are the known hydrogenation catalysts, e.g. those based on metals from group VIII of the periodic system, e.g. palladium, nickel, platinum, iridium or rhodium. The catalytic material may occur on a carrier, e.g. carbon, aluminium oxide, silica or titanium oxide. Particularly suitable as a catalyst in this method are Raney nickel and palladium-on-carbon. The catalysts are preferably used in quantities of 1—100 mg atom of active substance per kg benzyl benzoate, specifically in quantities of 2—50 mg atom of active substance per kg benzyl benzoate. In applying the method according to the invention generally 2—15 N litres hydrogen is taken up per kg benzyl benzoate and per hour. Often, 5—10 N liters hydrogen per kg benzyl benzoate is taken up per hour. The benzyl benzoate can be brought into contact with hydrogen in different ways. For example, the benzyl benzoate can be stirred in a hydrogen atmosphere for a certain length of time, or the hydrogen can be bubbled through the benzyl benzoate or be passed over it. The duration of the treatment of the benzyl benzoate with hydrogen is mostly between 0.25 and 5 hours, preferably between 0.5 and 1.5 hours. The use of larger quantities of catalyst, of a large excess of hydrogen, and/or the extension of the duration of the treatment are not excluded, but offer no advantages. This treatment of crude benzyl benzoate with hydrogen is effected at a temperature from 290 to 380 K. Particularly suitable are temperatures of between 320 and 360 K. The reaction pressure must be such that the liquid phase is maintained. A suitable reaction pressure is, e.g., between 100 and 1000 kPa, specifically between 200 and 500 kPa.

In the treatment of the benzyl benzoate with the above metals with reducing properties, these metals are mostly consumed in quantities of between 5 and 30 mg atom per kg benzyl benzoate, often in quantities of between 10 and 20 mg atom per kg benzyl benzoate. A suitable temperature range for this treatment of benzyl benzoate is from the freezing point of the water under the reaction circumstances (about 270 K) to 380 K, specifically from 275 to 360 K. The duration of the treatment is mostly between 0.1 and 5 hours, preferably between 0.5 and 3 hours. Suitable quantities of water during this treatment are, e.g., 1—1000 g per kg benzyl benzoate. Particularly suitable are quantities of 20—500 g per kg benzyl benzoate. The reaction pressure must be such that the liquid phase is maintained. A suitable reaction pressure is, e.g., between 50 and 300 kPa, specifically between 75 and 200 kPa.

In order to shorten the time required for the treatment and/or to lower the temperature required for the treatment, a base can be incorporated in the water. If in this process a sufficiently strong alkaline solution, e.g. 2—6 N, is used, washing with an aqueous alkaline solution, after the "metal-base" treatment, is virtually superfluous. Suitable bases are bases soluble in water, e.g. hydroxides of alkali metals and earth alkaline metals, the alkali metal carbonates, the alkali metal hydrogen carbonates, ammonia or organic bases soluble in water, e.g. amines. Particularly suitable are the hydroxides and carbonates of sodium and potassium, and calcium hydroxide. Suitable are quantities of base to 2500 mmole per kg benzyl benzoate, specifically to 1500 mmole per kg benzyl benzoate. The quantity of base is calculated as the equivalent quantity of NaOH.

Suitable aqueous alkaline solutions for the washing of the treated benzyl benzoate are, e.g. aqueous solutions of hydroxides of alkali metals and earth alkaline metals, alkali metal carbonates, alkali metal hydrogen carbonates, ammonia or organic bases such as amines. Particularly suitable are aqueous solutions of hydroxides or carbonates of sodium or potassium, and calcium hydroxide. In these washings quantities of solution of 0.1—0.5 litre per kg benzyl benzoate of a strength of 2—6 N are often used. The washing is often done at a temperature between the freezing point of the water under the prevailing conditions (about 270 K) and 330 K and at a pressure of 50—200 kPa. Instead of washing with an aqueous alkaline solution, the treated benzyl benzoate can be passed, if desired, over a basic ion exchanger, with which virtually the same results are achieved.

The distillation of the treated benzyl benzoate can be carried out under atmospheric or elevated, specifically, however, at reduced pressure, e.g. at a pressure of 0.5—5 kPa.

The saponification of the benzyl benzoate is carried out with the relative alkali metal hydroxide and/or suitable salts, e.g. sodium carbonate, potassium carbonate. Particularly suitable are aqueous solutions of preferably 2—4 N. The saponification is often carried out at a temperature of between 350 and 400 K and a pressure of 100—200 kPa.

According to a suitable mode of applying the method according to the invention, a mixture of the impure benzyl benzoate and water, optionally containing a base, is passed over a bed of solid metal particles. Particularly suitable for this purpose is a bed of zinc dust. No metal particles need then be separated, after the treatment, from the reaction mixture. Advantages of this procedure are the long on-stream time of the equipment now possible owing to the very slow exhaustion of the material present.

The invention is further elucidated by means of the following examples and of the comparative experiment.

Example I

75 g crude benzyl benzoate, which remained as a by-product after distillation of benzoic acid from a reaction mixture prepared by oxidation of toluene with a gas containing molecular oxygen and 0.375 g palladium-on-carbon (5% by weight of Pd), was heated in a reaction vessel of 500 ml at 323 K for 1.5 hours in the presence of hydrogen while being well shaken. The initial pressure was 445 kPa. After the treatment the pressure had fallen to 245 kPa. After the catalyst had been filtered off, the product was distilled. The main fraction was subsequently washed with cold 3 N aqueous NaOH. After removal of this alkaline water layer the remaining benzyl benzoate was saponified with aqueous NaOH to sodium benzoate and benzyl alcohol. From the resulting solution benzyl alcohol was extracted with toluene. The sodium benzoate was recovered by evaporation of the aqueous solution. Before the evaporation this solution was cleaned of residues of toluene, benzyl alcohol, etc., by steam distillation. The sodium benzoate obtained was found, in the above tests, to meet the stated requirements.

Example II

150 g crude benzyl benzoate, which remained as a by-product after distillation of benzoic acid from a reaction mixture prepared by oxidation of toluene with a gas containing molecular oxygen, and 75 ml water and 5 g zinc granules were heated for 1 hour at 353—358 K at atmospheric pressure, while being stirred. After this treatment the reaction mixture was cooled down and the zinc granules were separated out. After that the water layer was separated out. Subsequently the benzyl benzoate was washed with 75 ml 3 N aqueous NaOH at a temperature of 278—283 K. After removal of this alkaline water layer the remaining benzyl benzoate was distilled. The main fraction was subsequently saponified with aqueous NaOH to sodium benzoate and benzyl alcohol. From the resulting solution benzyl alcohol was extracted by means of toluene. The sodium benzoate was recovered from the aqueous solution in the same way as in example I. The sodium benzoate obtained was found, in the above tests, to meet the stated requirements.

Example III

120 g crude benzyl benzoate, which remained as a by-product after distillation of benzoic acid from a reaction mixture prepared by oxidation of toluene, with a gas containing molecular oxygen, and 12 ml 6 N aqueous NaOH and 4 g zinc granules, were heated at 313 K for 2.5 hours at atmospheric pressure, while being well stirred. After the alkaline water layer and the zinc had been separated out, the benzyl benzoate was distilled. The main fraction was subsequently saponified with aqueous NaOH to sodium benzoate and benzyl alcohol. From the resulting solution benzyl alcohol was extracted with toluene. The sodium benzoate was recovered from the aqueous solution in the same way as in example I. The sodium benzoate obtained was found, in the above tests, to meet the stated requirements.

Example IV

200 g crude benzyl benzoate, which remained as a by-product after distillation of benzoic acid from a reaction mixture prepared by oxidation of toluene, with a gas containing molecular oxygen, and 1.37 g aluminium powder, were cooled at 278—283 K. With good stirring, 90 ml 3 N aqueous NaOH was added in drops. The temperature was maintained at 278—283 K. The experiment was done at atmospheric pressure. After 0.5 hour the aluminium and the aqueous alkaline layer were separated out. After that the benzyl benzoate was distilled. The main fraction was subsequently saponified with aqueous NaOH to sodium benzoate and benzyl alcohol. From the resulting solution benzyl alcohol was extracted with toluene. The sodium benzoate was recovered from the aqueous solution in the same way as in example I. The sodium benzoate obtained was found, in the above tests, to meet the stated requirements.

Comparative experiment

106 g crude benzyl benzoate, which remained as a by-product after distillation of benzoic acid from a reaction mixture prepared by oxidation of toluene with a gas containing molecular oxygen was saponified with aqueous NaOH to sodium benzoate and benzyl alcohol. From the resulting solution benzyl alcohol was extracted by means of toluene. The sodium benzoate was recovered from the aqueous solution in the same way as in example 1. The sodium benzoate obtained was found, in the above tests, to be capable of meeting the stated requirements only in the acidity/alkalinity test.

Claims

1. Method for the preparation of an alkali metal benzoate together with benzyl alcohol, characterized in that crude benzyl benzoate, obtained as a by-product after distillation of benzoic acid from a reaction mixture prepared by oxidation of toluene with a gas containing molecular oxygen, is subjected to a treatment at 290—380 K with molecular hydrogen or at 270—380 K with a metal with reducing properties from the groups IA, IIA, IIB, IIIA, IVA, VIIB and VIII of the periodic system in the presence of water under such circumstances that there will be no conversion of any substantial quantity of benzyl benzoate and the purified benzyl benzoate is subsequently saponified to alkali metal benzoate and benzyl alcohol.

2. Method according to claim 1, characterized in that a catalyst is used containing a metal from group VIII of the periodic system.

3. Method according to claim 1, characterized in that the metal is applied as an aqueous suspension.

4. Method according to claim 1 or 3, characterized in that a base is incorporated in the water.

5. Method according to any one of the claims 1, 3 or 4, characterized in that a mixture of the impure benzyl benzoate and water, which may contain a base, is passed over a bed of solid metal particles.

6. Method according to any one of the claims 1—5, characterized in that the treated benzyl benzoate is washed with an aqueous alkaline solution.

7. Method according to any one of claims 1—6, characterized in that the reaction mixture, formed after the saponification of the treated benzyl benzoate, is extracted with an organic extractant, and the benzyl alcohol is subsequently recovered by distillation.

Revendications

1. Procédé de préparation d'un benzoate de métal alcalin avec l'alcool benzylique, caractérisé en ce qu'on soumet le benzoate de benzyle brut obtenu comme sous-produit après distillation d l'acide benzoïque à partir d'un mélange réactionnel préparé par oxydation du toluène avec un gaz contenant de l'oxygène moléculaire, à un traitement à 290—380°K avec de l'oxygène moléculaire ou à 270—380°K avec un métal ayant des propriétés réductrices des groupes IA, IIA, IIB, IIIA, IVA, VIIB et VIII de la classification périodique des élements en présence d'eau dans des circonstances telles qu'il n'y ait pas de transformation d'une quantité substantielle de benzoate de benzyle, puis en ce qu'on saponifie le benzoate de benzyle purifié en benzoate de métal alcalin et en alcool benzylique.

2. Procédé selon la revendication 1, caractérisé en ce qu'on utilise un catalyseur contenant un métal du groupe VIII de la classification périodique des éléments.

3. Procédé selon la revendication 1, caractérisé en ce que le métal est appliqué sous la forme d'une suspension aqueuse.

4. Procédé selon l'une des revendications 1 ou 3, caractérisé en ce qu'on incorpore une base dans l'eau.

5. Procédé selon l'une quelconque des revendications 1, 3 ou 4, caractérisé en ce qu'on fait passer un mélange de benzoate de benzyle impur et d'eau, qui peut contenir une base, sur un lit de particules métalliques solides.

6. Procédé selon l'une quelconque des revendications 1—5, caractérisé en ce qu'on lave avec une solution alcaline aqueuse le benzoate de benzyle traité.

7. Procédé selon l'une quelconque des revendications 1—6, caractérisé en ce qu'on extrait le mélange réactionnel formé après saponification du benzoate de benzyle traité, avec un produit d'extraction organique, puis en ce qu'on recueille par distillation l'alcool benzylique.

**Patentansprüche**

1. Verfahren zur Herstellung von Alkali-metallbenzoesäureester zusammen mit Benzyl-alkohol, dadurch gekennzeichnet, dass roher Benzoesäurebenzylester, der als Nebenprodukt nach der Destillation von Benzoesäure aus einem Reaktionsgemisch, hergestellt durch Oxydation von Toluol mit einem molekularen Sauerstoff enthaltenden Gas gewonnen wurde, einer Behandlung bei 290—380 K mit molekularem Wasserstoff oder bei 270—380 K mit einem Metall mit Reduktionseigenschaften aus den Gruppen IA, IIA, IIB, IIIA, IVA, VIIB und VIII des Periodensystems unterworfen wird, und zwar in Gegenwart von Wasser unter solchen Umständen, dass diese keine Umwandlung einer wesentlichen Menge des Benzoesäurebenzylesters bewirken und dass der gereinigte Benzoesäurebenzylester anschliessend zu Alkali-metallbenzoesäureeseter und Benzylalcohol ver-seift wird.

2. Verfahren nach Anspruch 1, dadurch gekennzeichnet, dass ein Katalysator verwendet wird, der ein Metall aus der Gruppe VIII des Perioden-systems enthält.

3. Verfahren nach Anspruch 1, dadurch gekennzeichnet, dass das Metall als wässrige Lösung verwendet wird.

4. Verfahren nach Anspruch 1 oder 3, dadurch gekennzeichnet, dass eine Base im Wasser enthalten ist.

5. Verfahren nach einem der Ansprüche 1, 3 oder 4, dadurch gekennzeichnet, dass ein Gemisch des unreinen Benzoesäurebenzylesters mit Wasser, welches eine Base enthalten darf über eine Lage fester Metallteile geführt wird.

6. Verfahren nach einem der Ansprüche 1—5, dadurch gekennzeichnet, dass der behandelte Benzoesäurebenzylester mit einer wässrigen alkalischen Lösung gewaschen wird.

7. Verfahren nach einem der Ansprüche 1—6, dadurch gekennzeichnet, dass das nach der Verseifung des behandelten Benzo-ebenzylsäureesters entstandene Reaktions-gemisch mit einem organischen Lösungsmittel extrahiert wird und dass der Benzylalkohol anschliessend durch Destillieren zurück-gewonnen wird.